# FASCICULE DE BREVET EUROPEEN

(11) **EP 1 582 222 B1**
(45) Date de publication et mention de la délivrance du brevet: **13.08.2008**
(21) Numéro de dépôt: 05300196.2
(22) Date de dépôt: 18.03.2005
(51) Int. Cl.: A61K 47/26, A61K 47/44, A61K 47/12, A61K 47/02, A61K 9/20

(54) **Tensioactifs sous forme de poudre utilisables dans des comprimés ou des gélules ; procédé de préparation et compositions les contenant**
Pulverförmige oberflächenaktive Stoffe zur Verwendung in Tabletten oder Kapseln, ihre Herstellungsverfahren und diese enthaltende Zubereitungen
Surface-active agents in powder form for use in tablets and capsules, their process of manufacture and compositions containing them

(30) Priorité: 29.03.2004 FR 0450614
(43) Date de publication de la demande: 05.10.2005
(73) Titulaire: SOCIETE D'EXPLOITATION DE PRODUITS POUR LES INDUSTRIES CHIMIQUES ( S.E.P.P.I.C.), 75321 Paris Cedex 07 (FR)
(72) Inventeur: Trouve, Gérard, 81100, CASTRES (FR); Aucouturier, Jérôme, 92290, CHATENAY-MALABRY (FR); Le Calvez, Guénolé, 94270, KREMLIN BICETRE (FR)
(74) Mandataire: Conan, Philippe Claude

(56) Documents cités:
- EP-A- 0 153 870
- EP-A- 0 639 638
- EP-A- 1 413 310
- DE-A- 19 913 606
- GB-A- 2 142 824

## Description

L'invention concerne de nouveaux tensioactifs en poudre leur procédé de préparation et leur application à la préparation de comprimés ou de gélules.

Les principes actifs pharmaceutiques développés aujourd'hui sont souvent des molécules complexes, peu solubles dans l'eau même en milieu acide. Lorsqu'ils sont administrés par voie orale, qu'il s'agisse principalement de comprimés ou de gélules, ces principes actifs se dissolvent difficilement en milieu gastrique ou intestinal, ce qui affecte leur bio - disponibilité, les concentrations plasmatiques nécessaires à l'effet thérapeutique recherché ne sont alors pas toujours atteintes et l'efficacité du médicament s'en donc trouve réduite.

Pour améliorer la solubilité et donc la bio - disponibilité de principes actifs peu solubles, on les formule parfois avec des tensioactifs compatibles avec une utilisation pharmaceutique ou alimentaire. Dans tout ce qui suit, on appellera « tensioactif » tout produit ou composition de produits susceptible, dans un domaine de concentration adéquat, d'abaisser la tension superficielle d'une solution aqueuse à une valeur inférieure à 50 mN/m, à température ambiante. La mesure de cette tension superficielle peut être faite par la méthode de la lame de Wilhemy. Parmi ces tensioactifs, certains ont un rôle de solubilisant qui permet de dissoudre une fraction significative du principe actif dans les fluides biologiques. On les trouve parmi les tensioactifs anioniques, comme par exemple le lauryl sulfate de sodium ou l'acide taurocholique ou encore parmi les tensioactifs non ioniques caractérisés par un nombre HLB (Hydrophilic Lipophilic Balance) supérieur à 12, souvent supérieur ou égal à 15, le nombre HLB étant calculé comme le rapport de la masse de la partie hydrophile du tensioactif à la masse molaire du tensioactif divisé par 5. Des exemples d'une telle utilisation de ces tensioactifs sont décrits dans : « Surfactant systems, chapitre 7, D. Attwood, A.T. Florence, Chapman & Hall éditeurs ». Ce livre de référence enseigne aussi que les tensioactifs solubilisants agissent en formant des agrégats plus ou moins sphériques, appelés micelles, à l'intérieur desquels se trouvent les molécules de principes actifs.

Cependant, de telles micelles ne se forment que lorsque la concentration en tensioactif en solution atteint une valeur minimale. Il est donc nécessaire d'incorporer des quantités importantes de tensioactif dans la dose médicamenteuse administrée au patient, pour que son effet solubilisant du principe actif s'exprime efficacement dans les milieux gastrique ou intestinal. Au chapitre 5 de ce même livre de référence cité ci-dessus, les valeurs présentées dans les tableaux mettent en évidence que les quantités de tensioactifs à mettre en oeuvre varient selon les principes actifs à solubiliser ; les rapports molaires entre le solubilisant et le principe actif qui y sont divulgués, sont compris entre 0, 25 et 1000. Dans la pratique, les rapports pondéraux entre le tensioactif solubilisant et le principe actif vont de 0,5 à 20, de préférence de 1 à 10.

Dans les formes galéniques solides comme les gélules et surtout les comprimés, la quantité nécessaire de tensioactif solubilisant à incorporer pour améliorer la solubilité et la bio - disponibilité du principe actif, ne doit toutefois pas altérer les propriétés galéniques et mécaniques de la formulation finale qui sont principalement la dureté, la vitesse de désagrégation, la vitesse d'écoulement ou la stabilité.

Les techniques modernes de fabrication de gélules ou de comprimés et en particulier la technique dite de compression directe, nécessitent de disposer d'excipients sous forme de solides finement divisés susceptibles de former avec les principes actifs un mélange de poudres aux propriétés physiques et mécaniques bien définies. En particulier, ce mélange doit s'écouler librement, c'est à dire que le temps d'écoulement de 100 g d'un tel mélange, mesuré selon le test 2-9-16 de la pharmacopée européenne, doit être inférieur à 10 s. Ce mélange doit aussi être comprimable, propriété qui peut être évaluée à partir de la mesure de l'aptitude au tassement décrite dans le test 2-9-15 de la pharmacopée européenne : la différence des volumes apparents du mélange après 10 et 500 tassements dans un appareil normalisé, doit ainsi être de préférence inférieure à 20 ml. Les comprimés obtenus doivent avoir des caractéristiques mécaniques acceptables, c'est à dire avoir une résistance à la rupture (encore appelée dureté) de préférence supérieure à 30 N, mesurée selon le protocole 2-9-8 de la pharmacopée européenne, et une friabilité mesurée selon le protocole 2-9-7 de la pharmacopée européenne, de préférence inférieure à 0,5 %.

Or la plupart des tensioactifs disponibles et utilisables pour des applications pharmaceutiques, ne répondent pas à ces exigences car ou bien ils sont liquides ou pâteux à température ordinaire ou bien il s'agit de solides à granulométrie très élevée, tels que des écailles par exemple. Le broyage de telles écailles sous forme de poudres fines est extrêmement difficile et coûteux, car les tensioactifs organiques ont des points de fusion souvent bas, de l'ordre de 50 °C à 100 °C et fondent sous l'effet de l'échauffement dans les broyeurs. De plus les rendements de broyage pour obtenir des poudres de quelques dizaines à quelques centaines de microns sont mauvais.

Le stéarate de magnésium est l'un des rares tensioactifs qui soit disponible sous forme de poudre fine, et qui soit incorporable directement dans un comprimé. Mais ce produit est essentiellement utilisé comme lubrifiant et il ne peut être utilisé à des concentrations élevées à cause de son effet néfaste sur la dureté du comprimé.

Pour obtenir des formes solides, notamment des comprimés, contenant un principe actif peu soluble dont la bio - disponibilité doit être améliorée, on prépare aujourd'hui des suspensions aqueuses ou des solutions dans des solvants organiques, du principe actif et du tensioactif, puis on pulvérise ces dispersions ou solutions sur des supports solides. Par exemple, la demande internationale publiée sous le numéro WO 90/01329 divulgue un procédé consistant en la préparation d'un mélange intime d'un polymère gastrorésistant, d'un tensioactif non ionique et d'un principe actif de nature protéique, dans un solvant organique tel que le méthanol, l'éthanol, l'acétone ou le chlorure de méthylène, puis en l'évaporation du solvant pour obtenir une poudre qui peut éventuellement être incorporée dans un comprimé. Mais l'utilisation de solvants organique limite la mise en oeuvre industrielle de ce procédé.

La demande de brevet européen EP 1 273 293 divulgue un procédé de préparation de FENOFIBRATE micronisé à dissolution améliorée qui consiste à préparer une suspension aqueuse de ce principe actif peu soluble en présence d'un polymère hydrophile et éventuellement d'un tensioactif, puis à pulvériser cette suspension sur un support solide hydrosoluble dans un lit fluidisé. Le granulé obtenu peut être comprimé ou introduit dans une gélule. L'amélioration de la solubilité et de la bio - disponibilité du FENOFIBRATE par ce procédé est donc liée à une forme particulière, micronisée, du principe actif et à un procédé de préparation spécifique en présence de polymère; elle n'est pas liée principalement au tensioactif, puisque sa présence est optionnelle. Un tel procédé est long et coûteux car il nécessite des équipements sophistiqués tels que des lits fluidisés ou des atomiseurs. La demande DE1999 13 606 A montre une composition pulvérulente pour la solubilisation d'un agent actif. Elle divulgue qu'une solution aqueuse contenant de la polyvinyl pyrrolidone et du Cremophor RH 40 (huile de ricin hydrogénée à 40 moles d'oxyde d'éthylène) est transformée en poudre par séchage par dispersion ; ladite poudre est incorporée dans un comprimé contenant de la cyclosporine.

La demande de brevet européen EP 0 639 638 A montre une pâte aqueuse de sodium alklyl (C 14, C15) sulphate et sodium alkyl (C13-C15) ester sulphate mélangée avec un mélange d'un sel de sodium d'un copolymère de l'acide maléique et acrylique avec de la silice pyrogénée ; ladite pâte étant granulée avec de la zéolite A et un carbonate. Les granule obtenus s'écoulenet librement.

La demande de brevet européen EP 10 153 870 A montre une composition tensioactive pulvérulente qui s'écoule librement, obtenue par extrusion à 130°C d'un mélange d'amidon de riz, d'eau et de lécithine pour l'utilisation dans l'industrie alimentiare. Le support solide présente des tailles de particules de 1 à 100 micromètres.

La demande WO 03/0060052, traduction dans EP 1 413 310 A, montre une composition pulvérulente contenant du mannitol ou de la cyclodextrine, du Tween 80 et un principe actif (« bone morphogenetic protein »), obtenue par séchage par dispersion.

Les inventeurs ont donc cherché à développer une méthode simple qui n'ait pas les inconvénients exposés ci-dessus, de préparation de compositions solides, ingérables à usage pharmaceutique, diététique, alimentaire ou cosmétique sous forme de comprimés, de gélules, de pâtes à mâcher, de granules ou de toute autre forme solide, ayant des propriétés mécaniques convenables, par simple mélange d'au moins un principe actif peu soluble dans l'eau et d'un tensioactif solubilisant permettant d'améliorer la bio - disponibilité du principe actif, suivi d'une étape de compression sans ajout de solvant.

Il a été trouvé que l'on pouvait résoudre le problème posé en préparant des compositions tensioactives caractérisées en ce qu'elles comprennent au moins un tensioactif liquide ou pâteux à température ambiante et un support solide de masse volumique apparente après tassement (pharmacopée européenne 2-9-15) inférieure à 0,5, sur lequel le ou les tensioactifs sont adsorbés. En choisissant judicieusement le support, on peut former des compositions tensioactives contenant de 1 % à 90 % d'un tensioactif liquide ou pâteux à température ambiante et de 99 % à 10 % d'un solide. Le tensioactif obtenu se présente sous forme d'une poudre ou d'un granulé de quelques dizaines à quelques centaines de microns de diamètre, qui possède un bon écoulement, une bonne aptitude à la compression directe ou à la granulation humide et qui est capable de libérer ledit tensioactif en présence d'un milieu biologique tel que le liquide gastrique ou le liquide intestinal. Une fois libéré ainsi à proximité du principe actif, le tensioactif peut jouer le rôle de solubilisant de principe actif.

C'est pourquoi, selon un premier aspect, l'invention a pour objet, une composition tensioactive (C_{T}) pulvérulente, caractérisée en ce qu'elle consiste essentiellement en un mélange de 1 % à 90 % en poids d'un tensioactif (TA) liquide ou pâteux à une température comprise entre 10°C et 35°C et de 10 % à 99 % en poids d'un support solide choisi parmi le phosphate de calcium ou les aluminosilicates, et en ce qu'elle s'écoule librement, le temps d'écoulement, mesuré par la méthode 2.9.16. de la Pharmacopée européenne 4^{ème} édition au moyen d'un entonnoir à écoulement normalisé décrit à la figure 2-9-16-2 de la Pharmacopée européenne, de 100 g de ladite composition C_{T}, étant inférieur ou égal à 10 s.

Comme tensioactif liquide ou pâteux à à une température comprise entre 10 °C et 35 °C, on désigne notamment mais de façon non limitative, les esters de sorbitan, les esters de sorbitan éthoxylés, les éthers de sucres, tels que les éthers de lactose, de saccharose, de xylose, de mannitol ou de xylitol, les alcools gras éthoxylés, les acides gras et leurs sels, les acides gras éthoxylés, les esters de polyglycérols, les copolymères d'oxyde de propylène et d'oxyde d'éthylène, les phospholipides ou lécithines, les acylâts à chaîne grasse d'acides aminés, les triglycérides d'origine végétale ou synthétique et leurs dérivés éthoxylés, les monoglycérides acétylés, le lauryl sulfate de sodium et ses dérivés ou encore l'acide taurocholique et ses dérivés.

Parmi les tensioactifs cités ci-dessus, il y a plus particulièrement :

Le monooléate de sorbitan eicosaéthoxylé (indice OE = 20 ; HLB = 15 ; liquide à température ambiante), commercialisé par exemple sous le nom de marque MONTA-NOX™ 80;

Le trioléate de sorbitan eicosaéthoxylé (indice OE = 20 ; HLB = 12 ; liquide à température ambiante), commercialisé par exemple sous le nom de marque MONTANOX™ 85 ;

Le monolaurate de sorbitan eicosaéthoxylé (indice OE = 20 ; HLB = 15 ; liquide à température ambiante), commercialisé par exemple sous le nom de marque MONTA-NOX™ 20 ;

L'alcool laurique tricosaéthoxylé (indice OE = 23 ; HLB = 16,9 ; cire à température ambiante), commercialisé par exemple sous le nom de marque SIMULSOL™ P23 ;

L'huile de ricin hydrogénée pentacosaéthoxylée (indice OE = 25 ; HLB = 12 ; liquide visqueux à température ambiante), commercialisé par exemple sous le nom de marque
SIMULSOL™ 1292 ;

L'huile de ricin hydrogénée tétracontaéthoxylée (indice OE = 40 ; HLB 14, liquide pâteux à température ambiante), commercialisé par exemple sous le nom de marque SI-MULSOL™ 4000 ou SIMULSOL™ 1293 ;

L'huile de ricin hexacontaéthoxylée (indice OE = 60 ; HLB = 14 ; liquide pâteux à température ambiante), commercialisé par exemple sous le nom de marque
SIMULSOL™ 1285 ;

L'acide oléique décaéthoxylée (indice OE = 10 ; HLB = 13 ; liquide à température ambiante), commercialisé par exemple sous le nom de marque SIMULSOL™ 2599.

On pourra utiliser aussi des mélanges de tous ces tensioactifs entre eux ou avec un tensioactif plus lipophile tel que le monooléate de sorbitan, (HLB = 4,3), commercialisé sous le nom de marque MONTANE™ 80.

Dans la composition tensioactive C_{T} telle que définie ci-dessus, le support solide a généralement une masse volumique apparente après tassement, inférieure à 0,5. Par masse volumique apparente, on désigne le rapport M / V dans lequel M représente la masse du matériau et V son volume apparent. La masse volumique apparente µ est déterminée selon le protocole expérimental 2 - 9 - 15 de la Pharmacopée européenne. Comme exemples d'un tel support solide, il y a ceux sous forme de poudres fines ou des granulés de quelques dizaines à quelques centaines de microns, solubles ou non en milieu aqueux, et qui présentent une grande surface spécifique. On peut citer par exemple le phosphate de calcium, ou les aluminosilicates. Parmi ces produits, on préfère ceux qui sont comprimables et de très grande surface spécifique, comme par exemple les phosphates de calcium commercialisés sous le nom de marque FUJICALIN™ ou les aluminosilicates de commercialisés sous le nom de marque NEUSILIN™.

Selon une autre caractéristique préférée, la composition (C_{T}) telle que définie ci-dessus, a une aptitude au tassement inférieure à 20 ml dans le test 2-9-15 de la Pharmacopée européenne.

Selon une dernière caractéristique préférée, la composition (C_{T}) telle que définie ci-dessus, présente des tailles de particules inférieures à 1000 µm, de préférence comprise entre environ 5 µm et environ 500 µm, de préférence encore entre environ 10 et environ 250 µm, mesurées en utilisant un granulomètre laser ou une série de tamis normalisés selon les prescriptions de la pharmacopée européenne 2-1-4.

Selon un deuxième aspect, l'invention a pour objet, un procédé de préparation de la composition tensioactive (C_{T}) telle que définie précédemment, caractérisé en ce que l'on adsorbe de 1 % à 80 % en poids d'un tensioactif liquide ou pâteux à une température comprise entre 10°C et 35°C sur de 20 % à 99 % en poids d'un support solide.

L'adsorption peut se faire par mélange dans un homogénéisateur mécanique tel que par exemple un mélangeur de marque DIOSNA™ ou Lodige™. Ce procédé est préféré si le support solide est insoluble dans l'eau, comme par exemple le phosphate de calcium. Dans ce cas, le tensioactif liquide est versé sous agitation dans le mélangeur préalablement chargé avec le support solide jusqu'à obtention d'un produit d'aspect sec et homogène. Elle peut aussi se faire par un procédé d'adsorption - granulation. Celui ci est avantageusement mis en oeuvre dans le cas de supports solides soluble tel que le lactose. On verse alors le tensioactif liquide sur le lactose jusqu'à obtention d'un mélange sec et homogène, puis on ajoute environ 2 % d'eau sur ce mélange pour obtenir un granulé de diamètre moyen environ 200 à 500 µm.

Elle peut encore se faire par pulvérisation du tensioactif liquide (pur ou en solution) sur le support solide dans un lit fluidisé, dans lequel un courant d'air chaud met en mouvement le support solide et élimine éventuellement le solvant dans lequel est dissout le tensioactif. Ce solvant est de préférence l'eau.

Dans ces différents procédés, il est possible de chauffer le tensioactif ou la solution de tensioactif que l'on souhaite adsorber sur le support solide, afin de diminuer la viscosité des liquides et d'en faciliter la répartition sur le support solide. Les proportions de tensioactifs pouvant être adsorbées sur les supports dépendent de la nature du support et de sa surface spécifique. Elles peuvent aller d'environ 1 à environ 95%, le plus souvent de 1 à 80% en poids, du produit final obtenu.

Pour améliorer encore la bio - disponibilité de l'actif, des cosolvants ou des hydrotropes, tels que par exemple du glycérol, des glycols, des huiles minérales ou végétales, des alcools légers... , peuvent être adsorbés conjointement aux tensioactifs sur le support solide.

Selon un troisième aspect, l'invention a pour objet une composition (C) comestible caractérisée en ce qu'elle comprend :
(a) - une quantité non nulle d'au moins un principe actif (PA),
(b) - une quantité non nulle et jusqu'à 80 % d'au moins une composition tensioactive (C_{T}) telle que définie précédemment et en ce que le rapport pondéral dans (C_{T}) / (PA) est supérieur ou égal à 0,25 et inférieur ou égal à 20 et de préférence supérieur ou égal à 1 et inférieur ou égal à 10, et éventuellement
(c) - jusqu'à 95 % en poids d'un ou plusieurs excipients comestibles,
   étant entendu que la somme des pourcentages pondéraux des composants (a) (b) et (c) est égale à 100 %.

Par excipient comestible, on désigne les excipients usuellement utilisés dans la préparation de forme galéniques destinée à l'administration orale. Pour les comprimés, on entend aussi bien les excipients du noyau de le formulation que les excipients d'enrobage dudit noyau. On cite plus particulièrement les diluants comme le lactose, les amidons, les celluloses microcristallines, les phosphates ou carbonate de calcium, les liants comme les alcools polyvinyliques, la povidone, les dérivés de la cellulose, les amidons prégélatinisés, les lubrifiants comme le stéarate de magnésium, l'acide stéarique, les huiles végétales hydrogénées les triglycérides de synthèse, le talc, les agents d'écoulement comme les silices, les désintégrants comme les carboxyméthylcelluloses , les povidones réticulées les agents mouillants comme les polysorbates, le laurylsulfate de sodium, les lécithines, les polymères filmogènes comme les polymères acryliques, les polymères cellulosiques, les alcools polyvyniques, les plastifiants comme la glycérine, les polyéthylène glycols, le propylène glycol, les monoglycérides acétylés, la triacétine, les phtalates, les agents colorants sous formes de laques ou de pigments comme les oxydes de fer ou de titane.

Par comestible on entend toute composition ingérable qu'il s'agisse de médicaments, de produits destinés à une application cosmétique ou de compléments alimentaires. Il peut aussi s'agir de produits de confiserie ou d'extraits de végétaux.

Selon une caractéristique particulière la composition (C) telle que définie précédemment contient jusqu'à 20 % en poids de principe actif (PA).

Selon une caractéristique particulière la composition (C) telle que définie précédemment contient jusqu'à 80 % en poids de la composition tensioactive (C_{T}) telle que définie précédemment.

Selon une autre caractéristique préférée, la composition (C) telle que définie ci-dessus, se présente sous forme d'un solide pulvérulent s'écoulant librement, le temps d'écoulement de 100 g de tensioactif étant inférieur à 10 s dans le test 2-9-16 de la Pharmacopée européenne.

Selon une autre caractéristique préférée, la composition (C) telle que définie ci-dessus, a une aptitude au tassement inférieure à 20 ml dans le test 2-9-15 de la Pharmacopée européenne.

Selon une dernière caractéristique préférée, la composition (C) telle que définie ci-dessus, présente des tailles de particules inférieures à 1000 µm, de préférence comprise entre environ 5 µm et environ 500 µm, de préférence encore entre environ 10 et environ 250 µm, mesurées en utilisant un granulomètre laser ou une série de tamis normalisés selon les prescriptions de la pharmacopée européenne 2-1-4.

La composition (C) telle que définie ci-dessus peut être mise en oeuvre plus particulièrement sous la forme de comprimés, de gélules, de pâtes à mâcher ou de granules

Selon un quatrième aspect, l'invention a pour objet, l'utilisation de la composition tensioactive (C_{T}) comme agent solubilisant d'un principe actif (PA).

Selon un dernier aspect, l'invention a pour objet, l'utilisation de la composition tensioactive (C_{T}) telle que définie, comme agent solubilisant dans des comprimés, des gélules, des pâtes à mâcher ou des confiseries.

Les exemples suivants illustrent l'invention sans toutefois la limiter.

### Préparation de compositions tensioactives selon l'invention

**Exemple 1 :** On prépare des tensioactifs sous forme de poudres par adsorption de différents tensioactifs liquides sur un support solide poreux, le phosphate de calcium FUJI-CALIN™ SG commercialisé par Fuji, Japon. Les différents tensioactifs utilisés successivement sont :
le MONTANOX™ 80, le SIMULSOL™ P23, le SIMULSOL™ 1292, le SIMULSOL™ 4000, le mélange MONTANE™ 80 / MONTANOX™ 80, en proportion pondérale 84 /16 et le mélange MONTANE™ 80 / MONTANOX™ 80 en proportion pondérale 65/35.
400 g de support solide sont introduits dans la cuve d'un mélangeur DIOSNA™ V10. 270g d'agent tensioactif préalablement réchauffé en chambre chaude si nécessaire, sont pesés puis introduits en continu à l'aide de l'entonnoir sur la poudre et sous agitation à 205 tours / minute.

Les caractéristiques des tensioactifs sous forme de poudre obtenus, sont déterminées selon les protocoles 2-9-16 de la pharmacopée européenne pour le temps d'écoulement, et 2-9-15 (volume apparent) pour l'aptitude au tassement.

### Détermination du temps d'écoulement

On introduit sans tasser, 100 g de poudre dans un entonnoir normalisé décrit à la figure 2-9-16-2 de la pharmacopée européenne dont l'orifice a été préalablement obturé. On libère l'orifice et mesure le temps d'écoulement de la totalité de l'échantillon. On effectue trois déterminations.

Le résultat consistant ou bien en la moyenne des trois mesures à conditions qu'aucune des valeurs individuelles ne s'écarte de plus de 10% de la valeur moyenne ou bien en la moyenne des deux valeurs extrêmes, si les valeurs individuelles s'écartent de plus de 10 % de la valeur moyenne, représente l'aptitude à l'écoulement de la poudre.

### Détermination du volume apparent.

Dans une éprouvette sèche, de 250 ml, graduée tous les 2 ml, de masse 220 ± 40 g, on verse 100 g de la poudre obtenue on l'on en mesure le volume apparent non tassé V₀ à 1 ml près. On fixe l'éprouvette sur le support de la machine à tasser de marque ERWEKA™, ledit support avec son dispositif de fixation ayant une masse de 450 ± 5 g et ladite machine pouvant provoquer par minute 250 ± 15 chutes d'une hauteur de 3 ± 0,2 mm.

On fait subir à la poudre, 10, 500 puis 1250 chutes en lisant respectivement les volumes après 10 chutes (V₁₀), après 500 chutes (V₅₀₀) et après 1250 chutes (V₁₂₅₀). Si la différence V₅₀₀ - V₁₂₅₀ est supérieure à 2 ml on fait subir 1250 nouvelles chutes et on mesure le volume après 2500 chutes (V₂₅₀₀).

Ces mesures permettent d'exprimer les résultats suivants :
- le volume apparent avant tassement ou volume vrac : V₀ ;
- le volume apparent après tassement ou volume tassé : V₁₂₅₀ (ou, le cas échéant : V₂₅₀₀);
- l'aptitude au tassement : V₁₀ -V₅₀₀ ;
- la masse volumique apparente avant tassement ou masse volumique du produit vrac : m/ V₀ ;
- la masse volumique apparente après tassement ou masse volumique du produit tassé : m/V₁₂₅₀ (ou, le cas échéant : m/V₂₅₀₀) ;

Il est généralement admis que les poudres présentant des temps d'écoulement inférieurs à 10 secondes et des aptitudes au tassement inférieures à 20 ml possèdent les qualités requises d'écoulement libre et de comprimabilité pour être utilisés dans la fabrication de comprimés ou gélules.

La granulométrie de la poudre obtenue est obtenue en en déterminant le diamètre moyen avec un granulomètre laser Mastersizer™ de Malvern.

Les résultats sont consignés dans le tableau suivant :

| **Tensioactif poudre** | **Ecoulement (s)** | **Aptitude au tassement (V₁₀ - V₅₀₀) en ml** | **Diamètre moyen (µm)** |
|---|---|---|---|
| FUJICALIN™ SG + MONTANOX™ 80 | 6.0 | 10 | 215 |
| FUJICALIN™ SG + SIMULSOL™ 1292 | 4,5 | 8 | 153 |
| FUJICALIN™ SG + SIMULSOL™ 4000 | 4,6 | 8 | 172 |
| FUJICALIN™ SG + SIMULSOL™ P23 | 3,1 | 5 | 133 |
| FUJICALIN™ SG + [MONTANE™ 80 - MONTANOX™ 80 (84/16)] | 4,3 | 7 | 186 |
| FUJICALIN™ SG + [MONTANE™ 80 - MONTANOX™ 80 (65/35)] | 4,5 | 8 | 179 |

**Exemple 2 :** On reproduit l'essai de l'exemple 1 en remplaçant le support solide FUJICALIN™ SG par un alumino - métasilicate de magnésium NEUSILIN™ US2 commercialisé par Fuji, Japon. Dans ce cas, 600g de chacun des tensioactifs initiaux sont mélangés avec 200g de NEUSILIN dans le mélangeur DIOSNA™ V10. Les tensioactifs sous forme de poudres obtenus présentent les caractéristiques suivantes :

| **Tensioactif poudre** | **Ecoulement (s)** | **Aptitude au tassement (V₁₀ - V₅₀₀) en ml** | **Diamètre moyen (µm)** |
|---|---|---|---|
| NEUSILIN™ + MONTANOX™ 80 | 8,2 | 7 | 95 |
| NEUSILIN™ + SIMULSOL™ 1292 | 8,6 | 7 | 100 |
| NEUSILIN™ + SIMULSOL™ 4000 | 8,5 | 9 | 103 |
| NEUSILIN™ + SIMULSOL™ P23 | 8,2 | 8 | 87 |
| NEUSILIN™ + [MONTANE™ 80-MONTANOX™ 80 (84/16)] | 9,8 | 8 | 90 |
| NEUSILIN™ + [MONTANE™ 80-MONTANOX™ 80 (65/35)] | 7,3 | 10 | 88 |

**Exemple 3 :** Dans un mélangeur de type DIOSNA™ V100, on introduit 10 kg d'un alumino - métasilicate de magnésium (NEUSILIN™ US2 - Fuji) sur lequel on verse progressivement 23,5 kg de polysorbate 80 (MONTANOX™ 80 - Seppic) au moyen d'une pompe, tout en maintenant une agitation à la vitesse 1 dans le mélangeur pendant 2 minutes à température ambiante. On obtient un tensioactif sous forme de poudre présentant les caractéristiques suivantes :

| | |
|---|---|
| Aptitude à l'écoulement | 7 s |
| Aptitude au tassement (V₁₀ - V₅₀₀) | 14 ml |
| Masse volumique après tassement | 0,59 g / ml |
| Refus au tamis de 200µm | 10 % |
| Diamètre moyen Dv50 (µm)(granulomètre laser) | 102 µm |

### Compositions tensioactives + principe actif) selon l'invention

**Exemple 4 :** Le Procetofène ou 2-[4-(4-chlorobenzoyl) phénoxy]-2-méthylpropionate d'isopropyle, commercialisé sous le nom de FENOFIBRATE™, est un principe actif inhibiteur de la synthèse hépatique du cholestérol et des glycérides plasmatiques. Il est pratiquement totalement insoluble dans l'eau (solubilité< 3 mg/l. On étudie les cinétiques de dissolution du FENOFIBRATE™ en utilisant un appareil Dissolutest™ DT600 ERWEKA, réglé à 37°C ± 0,5°C, avec une rotation des pâles de 200 tours / minute. Le milieu de dissolution est constitué de 500 ml d'un milieu tampon de pH 1,7 préparé conformément à la Pharmacopée européenne. Des prélèvements sont effectués régulièrement, puis sont ensuite filtrés à l'aide d'un filtre seringue et la quantité de FENOFI-BRATE™ dans ces prélèvements est déterminée par Chromatographie Liquide Haute performance (HPLC) munie d'un détecteur UV à 286 nm.

Dans une expérience préliminaire, on démontre la capacité du SIMULSOL™ 4000 à solubiliser le FENOFIBRATE™. Pour cela, on introduit 1500 mg de FENOFIBRATE™ et 1000 mg de SIMULSOL™ 4000 dans le tampon et on mesure les quantités de principe actif solubilisées au cours du temps. La quantité de FENOFIBRATE™, exprimée en % du FENOFIBRATE™ total introduit, augmente rapidement jusqu'à une valeur plateau de 1 % environ. On étudie ensuite les cinétiques de dissolution du FENOFIBRATE™ contenu dans des gélules de Lipanthyl™ 200, un médicament commercialisé sur le marché français. Le protocole mis en oeuvre est celui préconisé au chapitre 2-9-3 de la Pharmacopée européenne ; on utilise le même appareil Dissolutest ERWEKA™, dans les mêmes conditions que précédemment. Des prélèvements sont effectués après 5, 10, 20 et 30 minutes, filtrés, puis analysés par HPLC munie d'un détecteur UV à 286 nm. Les résultats ci dessous confirment la très faible solubilité du FENOFIBRATE™. Au bout de 30 min d'agitation, on introduit le tensioactif sous forme poudre fabriqué à partir de NEUSILIN™ et de SI-MIJLSOL™ 4000, dont la préparation est décrite dans l'exemple 3. La masse de tensioactif poudre introduite est égale à la masse de FENOFIBRATE™ contenue dans la gélule. En conséquence, le ratio SIMULSOL™ 400 / FENOFIBRATE™ est le même que celui mis en jeu dans l'expérience préliminaire. De nouveaux prélèvements sont effectués 5, 10, et 20 min après introduction du tensioactif sous forme de poudre. La quantité de FENOFI-BRATE™ augmente rapidement jusqu'à la même valeur plateau de 1 % que dans l'expérience préliminaire. L'effet solubilisant du tensioactif sous forme de poudre est ainsi démontré ; il est équivalent à celui de la forme initiale du tensioactif.

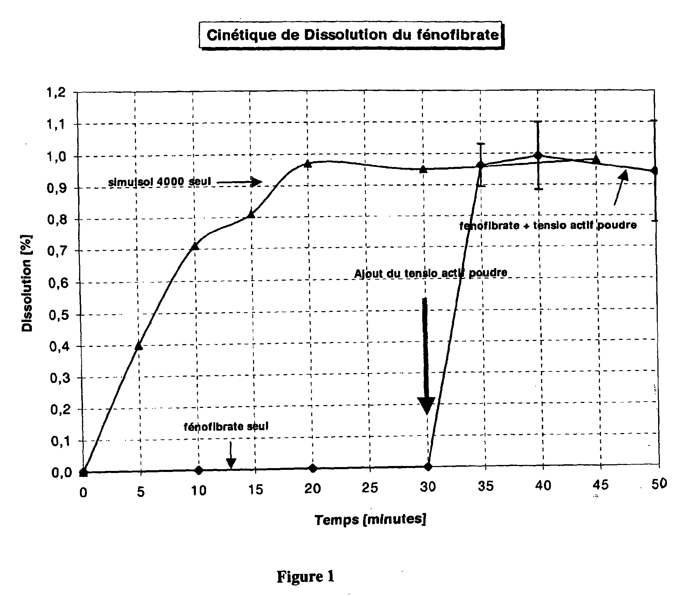

La figure 1 est un graphe mettant en évidence les résultats de la présente étude.

**Exemple 5 :** On étudie maintenant la dissolution de FENOFIBRATE™ contenu dans des comprimés. Outre le principe actif, les comprimés contiennent du lactose et de la cellulose micro cristalline comme diluants et liants respectivement, du stéarate de magnésium comme agent lubrifiant et le tensioactif sous forme poudre fabriqué à partir de NEU-SILIN™ et de SIMULSOL™ 4000, dont la préparation est décrite à l'exemple 2, comme solubilisant du FENOFIBRATE™. Trois séries de comprimés, avec des ratios tensioactifs poudre [C_{T}] / FENOFIBRATE [PA] différents, ainsi qu'une formule témoin, sont préparées. Pour fabriquer les comprimés, on mélange tout d'abord l'ensemble des poudres dans un mélangeur Turbula™ puis on les comprime au moyen d'une presse rotative Frogerais™ MR6, équipée de 6 poinçons de 11 cm de diamètre. La composition et les caractéristiques des comprimés obtenus, sont données dans le tableau ci dessous. Tous ont des friabilités et dureté acceptables.

### Détermination de la friabilité des comprimés

On place un échantillon de 20 comprimés sur un tamis n°1000 (1000 µm) et on élimine les poussières libres au moyen d'air comprimé. On pèse ensuite les comprimés puis on les place dans un tambour rotatif de marque ERWEKA™ de diamètre intérieur 290 mm, constitué d'un polymère synthétique transparent à surfaces intérieures polies ne produisant pas d'électricité statique, monté sur un système d'entraînement dont la vitesse de rotation est de 25 ± 1 tours par minute. L'on procède à 100 rotations, les comprimés sont sortis du tambour, libérés des poussières libres avec de l'air comprimé et pesés au mg près. Si la perte de masse est supérieure à 1 % l'opération est répétée deux nouvelles fois et le résultat est la moyenne des trois résultats. La friabilité est exprimée en termes de perte de masse et calculée en pourcentage de la masse initiale.

### Détermination de la dureté des comprimés

La dureté des comprimés est déterminée au moyen d'un appareil à deux mâchoires permettant la mesure de la résistance à la rupture du comprimé par écrasement entre les deux mâchoires. La mesure est effectuée sur 10 comprimés. Le résultat est la valeur moyenne des forces mesurées en Newton.

| | | Ratio [C_{T}] / [PA] | | | |
|---|---|---|---|---|---|
| Composants | Témoin | 1/2 | 1 | 2 | 10 |
| FENOFIBRATE™ | 15,00 % | 15,00 % | 15,00 % | 7,50 % | 1,50 % |
| Tensioactif poudre | 0,00 % | 7,50 % | 15,00 % | 15,00 % | 15,00 % |
| NEUSILIN™ | 15,00 % | 0,00 % | 0,00 % | 0,00 % | 0,00 % |
| Lactose FAST FLO™ | 52,125 % | 57,75 % | 52,125 % | 57,75 % | 62,25 % |
| VIVAPUR™ PH 102 | 17,375 % | 19,25 % | 17,375 % | 19,25 % | 20,75 % |
| Stéarate de Magnésium | 0,50 % | 0,50 % | 0,50 % | 0,50 % | 0,50 % |
| Poids (mg) | 547 | 537 | 558 | 563 | 544 |
| Friabilité (%) | 0,4 % | 0,43 % | 0 | 0,02 % | 0,4 % |
| Dureté (N) | 32 | 48 | 66 | 61 | 36 |

Les cinétiques de dissolution des comprimés préparés précédemment, sont réalisées conformément au protocole 2-9-3 de la Pharmacopée européenne.

Un appareil Dissolutest™ ERWEKA™ réglé à 37°C ± 0,5°C avec une rotation des pâles de 200 tr/min est utilisé. Les milieux de dissolution sont des milieux tampon de pH 1,7 et 7,2 préparés conformément à la Pharmacopée européenne, de volume 500ml. Des prélèvements sont effectués après 5, 10, 15, 20, 30 et 45 minutes. Ces prélèvements sont ensuite filtrés à l'aide d'un filtre seringue puis analysés par HPLC munie d'un détecteur UV à 286 nm. Les résultats des dosages sont exprimés en pourcentages de la quantité de FENOFIBRATE introduite. Les tableaux ci-dessous, montre l'évolution de ce pourcentage au cours du temps. Il met en évidence une très significative augmentation du pourcentage de FENOFIBRATE dissout par rapport à l'essai témoin quand on augmente la quantité de tensioactif selon l'invention dans les comprimés, quel que soit le pH.

| | | **Ratio [C_{T}] / [PA]** | | | |
|---|---|---|---|---|---|
| | **Témoin** | **1/2** | **1** | **2** | **10** |
| 5 min | - | 0,15 | 0,15 | 3,69 | 11,9 |
| 10 min | 0,015 | 0,20 | 0,53 | 3,43 | 12,8 |
| 15 min | - | 0,23 | 0,82 | 3,09 | 12,7 |
| 20 min | 0,015 | 0,18 | 1,33 | 2,81 | 11,5 |
| 30 min | 0,015 | 0,18 | 1,46 | 2,44 | 12,8 |
| 45 min | 0,015 | 0,20 | 1,55 | 2,33 | 12,6 |

| | | | | | |
|---|---|---|---|---|---|
| % de la quantité maximale de FENOFIBRATE solubilisable- Milieu tampon pH 1,7 | | | | | |

| | | **Ratio [C_{T}] / [PA]** | | | |
|---|---|---|---|---|---|
| | **Témoin** | **1/2** | **1** | **2** | **10** |
| 5 min | - | 0,32 | 0,02 | 4,26 | 22,4 |
| 10 min | 0,015 | 0,48 | 0,13 | 4,16 | 23,7 |
| 15 min | - | 0,52 | 0,66 | 3,79 | 25,2 |
| 20 min | 0,015 | 0,50 | 1,20 | 3,52 | 23,8 |
| 30 min | 0,015 | 0,50 | 1,39 | 3,22 | 23,0 |
| 45 min | 0,015 | 0,48 | 1,45 | 2,96 | 20,9 |

| | | | | | |
|---|---|---|---|---|---|
| % de la quantité maximale de FENOFIBRATE solubilisable- Milieu tampon pH 7,2 | | | | | |

**Exemple 6 :** On reproduit l'expérience décrite à l'exemple 7 mais en utilisant le tensioactif sous forme de poudre constitué de NEUSILIN™ et de MONTANOX 80™, dont la fabrication est décrite à l'exemple 3. La composition des comprimés préparés et leurs caractéristiques, sont données ci dessous :

| | Comprimé 8 - 1 **Ratio 1/1 [C_{T}] / PA** | Comprimé 8 - 2 **Ratio 10/1 [C_{T}] / PA** |
|---|---|---|
| FENOFIBRATE : | 15 | 1,5 |
| Lactose | 51,4 | 61,5 |
| Cellulose microcristalline | 17,1 | 20,5 |
| Stéarate de magnésium | 0,5 | 0,5 |
| Acide stéarique | 1,0 | 1,0 |
| Tensioactif poudre de l'exemple 4. | 15 | 15 |
| Poids moyen (mg) | 542 | 533 |
| Dureté (N) | 38 | 45 |
| Friabilité (%) | 0,06 | 0,1 |

Leur dissolution est étudiée dans deux milieux tampons à pH 1,7 et 7,2 respectivement, en utilisant le protocole décrit dans l'exemple 5. Les résultats obtenus figurent dans le tableau ci dessous et montrent que le tensioactif sous forme de poudre permet une dissolution nettement améliorée par rapport à un comprimé témoin sans tensioactif poudre.

| | Témoin | [C_{T}] / [PA] = 1 | [C_{T}] / [PA] = 10 |
|---|---|---|---|
| 5 min | - | 0 | 13.4 |
| 10 min | 0.015 | 0,06 | 32.8 |
| 15 min | - | 0,15 | 47.5 |
| 20 min | 0.015 | 0,39 | 46.7 |
| 30 min | 0.015 | 0,66 | 41.5 |
| 45 min | 0.015 | 0,70 | 22.8 |

| | | | |
|---|---|---|---|
| % de la quantité maximale de FENOFIBRATE solubilisable - Milieu tampon pH 1,7 | | | |

| | Témoin | [C_{T}] / [PA] = 1 | [C_{T}] / [PA] = 10 |
|---|---|---|---|
| 5 min | - | 0,05 | 11,6 |
| 10 min | 0,015 | 0,06 | 27,0 |
| 15 min | - | 0,29 | 31,7 |
| 20 min | 0,015 | 0,53 | 46,4 |
| 30 min | 0,015 | 0,70 | 12,1 |
| 45 min | 0,015 | 0,76 | 11,8 |

| | | | |
|---|---|---|---|
| % de la quantité maximale de FENOFIBRATE solubilisable- Milieu tampon pH 7,2 | | | |

### Exemple 7 : comprimés de théophylline

La théophylline est un antiasthmatique peu soluble dans l'eau (solubilité maximale 8 mg / 1). On cherche à fabriquer un comprimé de théophylline à bio - disponibilité améliorée. Pour cela on réalise les mélanges de poudres décrits dans le tableau ci dessous en utilisant soit le tensioactif composé de FUJICALIN™ et de MONTANOX™ 80 dont la préparation est décrite dans l'exemple 1, soit le tensioactif composé de NEUSILIN™ et de MONTANOX™ 80 dont la préparation est décrite dans l'exemple 4. Les comprimés sont réalisés comme précédemment sur une machine Frogerais™ MR6. Leurs caractéristiques sont données dans le tableau ci dessous :

| | Comprimé 9-1 | Comprimé 9-2 | Référence |
|---|---|---|---|
| Théophylline anhydre | 10 | 10 | 10 |
| Lactose FAST FLO™ | 25,8 | 29 | 29 |
| Cellulose microcristalline | 38,7 | 43,5 | 43,5 |
| FUJICALIN™ -MONTANOX™ 80 | 25 | 0 | 0 |
| NEUSILIN™-MONTANOX™ 80 | 0 | 17 | 0 |
| Stéarate de magnésium | 0,5 | 0,5 | 0,5 |
| NEUSILIN™ | 0 | 0 | 17 |
| Poids moyen (mg) | 451 | 445 | 450 |
| Dureté (N) | 40 | 61 | 58 |
| Friabilité (%) | 0,06 | 0,03 | 0,08 |

Les essais de dissolution sont réalisés dans un milieu tampon acétate pH = 4,6, correspondant au pH du duodénum dans le tractus gastro-intestinal, préparé conformément à la Pharmacopée européenne 4.02. Les résultats, exprimés en % de la quantité totale de théophylline, montrent que les tensioactifs poudre incorporés dans les comprimés permettent la solubilisation d'une plus grande quantité de théophylline, avec des cinétiques qui dépendent de la nature du tensioactif en poudre.

| | **Comprimé 9-1** | **Comprimé 9-2** | **Référence** |
|---|---|---|---|
| 5 min | 10 | 10 | 32 |
| 10 min | 42 | 21 | 42 |
| 20 min | 66 | 59 | 55 |

| | **Comprimé 9-1** | **Comprimé 9-2** | **Référence** |
|---|---|---|---|
| 30 min | 70 | 75 | 62 |
| 40 min | 73 | 84 | 65 |
| 50 min | 73 | 89 | 69 |
| 60 min | 75 | 91 | 70 |

| | | | |
|---|---|---|---|
| Cinétique de dissolution de la Théophylline en milieu pH 4,6 | | | |

## Revendications

1. Composition tensioactive (C_{T}) pulvérulente, **caractérisée en ce qu'**elle consiste essentiellement en un mélange de 1 % à 90 % en poids d'un tensioactif (TA) liquide ou pâteux à une température comprise entre 10°C et 35°C et de 10 % à 99 % en poids d'un support solide choisi parmi le phosphate de calcium ou les aluminosilicates , et **en ce qu'**elle s'écoule librement, le temps d'écoulement mesuré par la méthode 2.9.16. de la Pharmacopée européenne au moyen d'un entonnoir à écoulement, de 100 g de ladite composition C_{T} étant inférieur ou égal à 10 s.

2. Composition tensioactive (C_{T}) telle que définie à la revendication 1 dans laquelle le tensioactif est choisi parmi les esters de sorbitan, les esters de sorbitan éthoxylés, les éthers de sucres, tels que les éthers de lactose, de saccharose, de xylose, de mannitol ou de xylitol, les alcools gras éthoxylés, les acides gras et leurs sels, les acides gras éthoxylés, les esters de polyglycérols, les copolymères d'oxyde de propylène et d'oxyde d'éthylène, les phospholipides ou lécithines, les acylâts à chaîne grasse d'acides aminés, les triglycérides d'origine végétale ou synthétique et leurs dérivés éthoxylés, les monoglycérides acétylés, le lauryl sulfate de sodium et ses dérivés ou l'acide taurocholique et ses dérivés.

3. Composition tensioactive (C_{T}) telle que définie à la revendication 2 dans laquelle le tensioactif est choisi parmi :
Le monooléate de sorbitan eicosaéthoxylé ;
Le trioléate de sorbitan eicosaéthoxylé ;
Le monolaurate de sorbitan eicosaéthoxylé ;
L'huile de ricin hydrogénée pentacosaéthoxylée ;
L'huile de ricin hydrogénée tétracontaéthoxylée ;
L'huile de ricin hexacontaéthoxylée;
L'acide oléique décaéthoxylée.
ou un mélange de l'un ou de plusieurs de ces tensioactifs entre eux avec le monooléate de sorbitan.

4. Composition tensioactive (C_{T}), telle que définie à l'une des revendications 1 à 3 dans laquelle le support solide a une masse volumique apparente après tassement, inférieure à 0,5.

5. Composition tensioactive (C_{T}), telle que définie à l'une des revendications 1 à 4, **caractérisée en ce qu'**elle présente une aptitude au tassement inférieure à 20 ml.

6. Composition tensioactive (C_{T}), telle que définie à l'une des revendications 1 à 5, **caractérisée en ce qu'**elle présente des tailles de particules inférieures à 1000 µm, plus particulièrement comprise entre 5 µm et 500 µm et de préférence encore 10 µm et 250 µm.

7. Procédé de préparation de la composition tensioactive (C_{T}) telle que définie à l'une des revendications 1 à 6, **caractérisé en ce que** l'on adsorbe de 1 % à 90 % en poids de tensioactif liquide ou pâteux à une température comprise entre 10°C et 35°C sur de 10 % à 99 % en poids de support solide.

8. Composition (C) comestible **caractérisée en ce qu'**elle comprend :
(a) - une quantité non nulle d'au moins un principe actif (PA),
(b) - une quantité non nulle et jusqu'à 80 % d'au moins une composition tensioactive (C_{T}) telle que définie à l'une des revendications 1 à 6, et **en ce que** le rapport pondéral dans (C_{T}) / (PA) est supérieur ou égal à 0,25 et inférieur ou égal à 20 et de préférence supérieur ou égal à 1 et inférieur ou égal à 10, et éventuellement
(c) - jusqu'à 95 % en poids d'un ou plusieurs excipients comestibles, étant entendu que la somme des pourcentages pondéraux des composants (a) (b) et (c) est égale à 100 %.

9. Composition telle que définie à la revendication 8, **caractérisée en ce qu'**elle contient jusqu'à 20 % en poids de principe actif (PA).

10. Composition telle que définie à l'une des revendications 8 ou 9, **caractérisée en ce qu'**elle contient jusqu'à 80 % en poids de la composition tensioactive (C_{T}).

11. Composition telle que définie à l'une des revendications 8 à 10, **caractérisée en ce qu'**elle se présente sous forme d'un solide pulvérulent s'écoulant librement, le temps d'écoulement de 100 g de tensioactif étant inférieur à 10 s..

12. Utilisation de la composition tensioactive (C_{T}) telle que définie à l'une des revendications 1 à 6, comme agent solubilisant d'un principe actif (PA).

13. Utilisation de la composition tensioactive (C_{T}) telle que définie à l'une des revendications 1 à 6, comme agent solubilisant dans des comprimés, des gélules, des pâtes à mâcher ou des confiseries.

## Claims

1. Pulverulent surfactant composition (Cₛ), **characterized in that** it consists essentially of a mixture of 1% to 90% by weight of a surfactant (SA) that is liquid or pasty at a temperature between 10°C and 35°C, and of 10% to 99% by weight of a solid support chosen from calcium phosphate and aluminosilicates, and **in that** it flows freely, the flow time, measured by method 2.9.16. of the European pharmacopoeia, by means of a standardized flow funnel, of 100 g of said composition Cₛ being less than or equal to 10 s.

2. Surfactant composition (C_{S}) as defined in Claim 1, in which the surfactant is chosen from sorbitan esters, ethoxylated sorbitan esters, ethers of sugars, such as ethers of lactose, of sucrose, of xylose, of mannitol or of xylitol, ethoxylated fatty alcohols, fatty acids and their salts, ethoxylated fatty acids, polyglyceryl esters, copolymers of propylene oxide and of ethylene oxide, phospholipids or lecithins, amino acid fatty chain acylates, triglycerides of plant or synthetic origin and their ethoxylated derivatives, acetylated monoglycerides, sodium lauryl sulphate and its derivatives, or taurocholic acid and its derivatives.

3. Surfactant composition (C_{S}) as defined in Claim 2, in which the surfactant is chosen from:
eicosaethoxylated sorbitan monooleate;
eicosaethoxylated sorbitan trioleate;
eicosaethoxylated sorbitan monolaurate;
pentacosaethoxylated hydrogenated castor oil;
tetracontaethoxylated hydrogenated castor oil;
hexacontaethoxylated castor oil;
decaethoxylated oleic acid,
or a mixture of one or more of these surfactants with one another or with sorbitan monooleate.

4. Surfactant composition (Cₛ) as defined in one of Claims 1 to 3, in which the solid support has an apparent density after tapping of less than 0.5.

5. Surfactant composition (Cₛ) as defined in one of Claims 1 to 4, **characterized in that** it has a tapping capacity of less than 20 ml.

6. Surfactant composition (Cₛ) as defined in one of Claims 1 to 5, **characterized in that** it has particle sizes of less than 1000 *µ*m, more particularly of between 5 *µ*m and 500 *µ*m, and more preferably of between 10 *µ*m and 250 *µ*m.

7. Process for preparing the surfactant composition (Cₛ) as defined in one of Claims 1 to 6, **characterized in that** from 1% to 90% by weight of surfactant that is liquid or pasty at a temperature between 10°C and 35°C is adsorbed onto from 10% to 99% by weight of solid support.

8. Edible composition (C), **characterized in that** it comprises:
(a) - a non-zero amount of at least one active principle (AP),
(b) - a non-zero amount and up to 80% of at least one surfactant composition (Cₛ) as defined in one of Claims 1 to 6, and such that the (Cₛ) / (AP) weight ratio is greater than or equal to 0.25 and less than or equal to 20, and preferably greater than or equal to 1 and less than or equal to 10,
and, optionally,
(c) - up to 95% by weight of one or more edible excipients,
it being understood that the sum of the percentages by weight of the components (a), (b) and (c) is equal to 100%.

9. Composition as defined in Claim 8, **characterized in that** it contains up to 20% by weight of active principle (AP).

10. Composition as defined in either of Claims 8 and 9, **characterized in that** it contains up to 80% by weight of the surfactant composition (Cₛ).

11. Composition as defined in one of Claims 8 to 10, **characterized in that** it is in the form of a free-flowing pulverulent solid, the flow time for 100 g of surfactant being less than 10 s.

12. Use of the surfactant composition (C_{S}) as defined in one of Claims 1 to 6, as an agent for solubilizing an active principle (AP).

13. Use of the surfactant composition (C_{S}) as defined in one of Claims 1 to 6, as a solubilizing agent in tablets, gelatin capsules, chewing gums or confectionery products.

## Patentansprüche

1. Pulverförmige oberflächenaktive Zusammensetzung (C_{T}), **dadurch gekennzeichnet, dass** sie im Wesentlichen aus einem Gemisch aus 1 Gew.-% bis 90 Gew.-% eines bei einer Temperatur im Bereich zwischen 10° C und 35 °C flüssigen oder pastenförmigen oberflächenaktiven Stoffs (TA) und 10 Gew.-% bis 99 Gew.-% eines festen Trägers, ausgewählt aus Calciumphosphat oder Aluminiumsilicaten besteht, und **dadurch**, dass sie frei fließt, wobei die Durchflusszeit, gemessen mit der Methode 2.9.16. des Europäischen Arzneibuchs mittels eines Abflusstrichters, von 100 g der Zusammensetzung C_{T} kleiner oder gleich 10 s ist.

2. Oberflächenaktive Zusammensetzung (C_{T}) wie in Anspruch 1 definiert, wobei der oberflächenaktive Stoff ausgewählt ist aus Sorbitanestern, ethoxylierten Sorbitanestern, Zuckerethern, wie etwa Lactose-, Saccharose-, Xylose-, Mannitol- oder Xylitolethern, ethoxylierten Fettalkoholen, Fettsäuren und ihren Salzen, ethoxylierten Fettsäuren, Polyglycerolestern, Propylenoxidcopolymeren und Ethylenoxidcopolymeren, Phospholipiden oder Lecithinen, Acylaten mit Aminosäurenfettkette, Triglyceriden pflanzlichen oder synthetischen Ursprungs und ihren ethoxylierten Derivaten, acetylierten Monoglyceriden, Natriumlaurylsulfat und seinen Derivaten oder Taurocholsäure und ihren Derivaten.

3. Oberflächenaktive Zusammensetzung (C_{T}) wie in Anspruch 2 definiert, wobei der oberflächenaktive Stoff ausgewählt ist aus:
eicosaethoxyliertem Sorbitanmonooleat;
eicosaethoxyliertem Sorbitantrioleat;
eicosaethoxyliertem Sorbitanmonolaurat;
pentacontaethoxyliertem hydriertem Rizinusöl;
Tetracontaethoxyliertem hydriertem Rizinusöl;
Hexacontaethoxyliertem Rizinusöl;
decaethoxylierter Ölsäure.
oder einem Gemisch aus einem oder mehreren dieser oberflächenaktiven Stoffe untereinander mit dem Sorbitanmonooleat.

4. Oberflächenaktive Zusammensetzung (C_{T}) wie in einem der Ansprüche 1 bis 3 definiert, wobei der feste Träger nach der Kompaktierung eine scheinbare Dichte kleiner als 0,5 hat.

5. Oberflächenaktive Zusammensetzung (C_{T}) wie in einem der Ansprüche 1 bis 4 definiert, **dadurch gekennzeichnet, dass** sie eine Kompaktierungsfähigkeit kleiner als 20 ml aufweist.

6. Oberflächenaktive Zusammensetzung (C_{T}) wie in einem der Ansprüche 1 bis 5 definiert, **dadurch gekennzeichnet, dass** sie Teilchengrößen kleiner als 1.000 µm, insbesondere im Bereich zwischen 5 µm und 500 µm und stärker bevorzugt zwischen 10 µm und 250 µm aufweist.

7. Verfahren zur Herstellung der oberflächenaktiven Zusammensetzung (C_{T}) wie in einem der Ansprüche 1 bis 6 definiert, **dadurch gekennzeichnet, dass** 1 Gew.-% bis 90 Gew.-% des bei einer Temperatur im Bereich zwischen 10° C und 35 °C flüssigen oder pastenförmigen oberflächenaktiven Stoffs auf 10 Gew.-% bis 99 Gew.-% festen Träger adsorbiert wird.

8. Essbare Zusammensetzung (C), **dadurch gekennzeichnet, dass** sie umfasst:
(a) - eine Menge von nicht null mindestens eines Wirkstoffs (PA),
(b) - eine Menge von nicht null bis zu 80 % mindestens einer oberflächenaktiven Zusammensetzung (C_{T}), wie in einem der Ansprüche 1 bis 6 definiert, und **dadurch**, dass das Gewichtsverhältnis in (C_{T}) / (PA) größer oder gleich 0,25 und kleiner oder gleich 20 und bevorzugt größer oder gleich 1 und kleiner oder gleich 10 ist, und gegebenenfalls
(c) - bis zu 95 Gew.-% eines oder mehrerer essbarer Hilfsstoffe,
wobei es sich versteht, dass die Summe der Gewichtsprozentanteile der Bestandteile (a), (b) und (c) gleich 100 % ist.

9. Zusammensetzung wie in Anspruch 8 definiert, **dadurch gekennzeichnet, dass** sie bis zu 20 Gew.-% des Wirkstoffs (PA) enthält.

10. Zusammensetzung wie in einem der Ansprüche 8 oder 9 definiert, **dadurch gekennzeichnet, dass** sie bis zu 80 Gew.-% der oberflächenaktiven Zusammensetzung (C_{T}) enthält.

11. Zusammensetzung wie in einem der Ansprüche 8 bis 10 definiert, **dadurch gekennzeichnet, dass** sie die Form eines pulverförmigen Feststoffs aufweist, der frei fließt, wobei die Durchflusszeit von 100 g des oberflächenaktiven Stoffs kleiner als 10 s ist.

12. Verwendung der oberflächenaktiven Zusammensetzung (C_{T}) wie in einem der Ansprüche 1 bis 6 definiert, als Lösungsvermittler eines Wirkstoffs (PA).

13. Verwendung der oberflächenaktiven Zusammensetzung (C_{T}) wie in einem der Ansprüche 1 bis 6 definiert, als Lösungsvermittler in Tabletten, Kapseln, Kaugummis oder Süßwaren.
